# EUROPEAN PATENT SPECIFICATION

(11) **EP 0 420 914 B1**
(45) Date of publication and mention of the grant of the patent: **09.03.1994**
(21) Application number: 89907586.5
(22) Date of filing: 14.06.1989
(51) Int. Cl.: A61K 7/48, A61K 37/02, C07K 5/08

(54) **COSMETIC AND SKIN TREATMENT COMPOSITIONS**
KOSMETISCHE UND HAUTBEHANDLUNGS-ZUSAMMENSETZUNGEN
PREPARATIONS COSMETIQUES ET POUR LES SOINS DE LA PEAU

(30) Priority: 16.06.1988 US 207698
(43) Date of publication of application: 10.04.1991
(73) Proprietor: PROCYTE CORPORATION, Kirkland, WA 98034-6900 (US)
(72) Inventor: PICKART, Loren, R., Bellevue, WA 98006 (US)
(74) Representative: Brown, John David
(86) International application number: US8902590
(87) International publication number: WO8912441

(56) References cited:
- EP-A- 0 189 182
- EP-A- 0 190 736
- EP-A- 0 288 278
- WO-A-88/08695
- WO-A-88/08851
- Chemical Abstracts, volume 106, 1987, (Colombus, Ohio, US), L. Pickart et al.:"Gly-L-His-L-Lys:copper(II) - a human plasma growth factor with superoxide dismutase-like and wound-healing properties", abstact 13579c, Superoxide Superoxide Dismutase Chem., Biol. Med., Proc. Int. Conf., 4th 1985 (Pub. 1986), 555-7

## Description

### Technical Field

The present invention relates to cosmetic compositions in general, and more specifically, to the use of derivatives of glycyl-L-histidyl-L-lysine: copper(II) (GHL-Cu) within skin treatment and cosmetic compositions.

### Background of the Invention

Skin problems in individuals can result from a variety of causes: environmental assault (e.g., sun and wind), internal disease (e.g., diabetes, atherosclerosis) or normal aging. A number of structural and functional skin changes occur with aging. Further, because of the interrelationship between the structure and function of the skin, structural changes resulting from the aging process may also lead to concomitant functional impairment.

Age-associated changes are readily apparent in the epidermis, where there is an increased propensity for blistering and/or erosion. Microscopically, it has been observed that the epidermal basal cells of aged skin display greater variability in their size, shape and staining qualities than those obtained from more youthful skin. In addition, the moisture content of the stratum corneum is decreased, and cellular cohesion is diminished, particularly at the periphery of the corneocytes.

Clinically, the problem of rough or dry skin is a manifestation of several morphological changes, including the decreased moisture content of the stratum corneum, coupled with reduced eccrine and sebaceous gland output. As a person ages, there is a decrease in the epidermal turnover time, especially after the age of 50. Clinically, superficial wounds take more time to heal, making the elderly more prone to secondary infection following minor trauma.

As the skin ages, the dermis decreases in density and becomes relatively acellular and avascular. Throughout adult life, the total amount of collagen decreases about one percent per year. The collagen fibers thicken, becoming less soluble, have less capacity for swelling, and become more resistant to digestion by collagenase. There are also structural aberrations in the elastic fibers of the reticular dermis that contribute to skin sagging and a predisposition to injury.

The regression of the subepidermal elastic network may contribute to cutaneous laxity and the subtle wrinkled appearance prevalent on sun-protected skin of the elderly. Atrophy of the dermis and subcutaneous fat also plays an important role in the formation of wrinkles.

In addition, the dermis of elderly individuals has approximately 50% fewer mast cells than does that of a younger person. Clinically, this has cosmetic as well as physiologic implications. Cosmetically, the skin becomes pale with advancing age. Physiologically, the elderly patient is predisposed to both hyperthermia and hypothermia, following seemingly insignificant changes in ambient temperature. Basically, a smaller volume of blood can be diverted to the reduced capillary network of the papillary dermis following elevations in the body's core temperature, thereby diminishing cooling and resulting in hyperthermia.

Conversely, hypothermia results from the body's inability to efficiently divert blood from the skin to help conserve body heat when ambient temperatures decrease. This problem is compounded by the loss of insulating subcutaneous tissue that generally occurs in the elderly.

Many preparations have been developed for the purpose of treating human skin in an effort to counter the structural changes briefly described above, or merely to temporarily enhance the appearance of the skin. Many such preparations are directed toward moisturizing, thereby protecting the skin against drying. In general, numerous cosmetic preparations intended to combat aging in the skin exist on the market, and these preparations contain a wide variety of compounds, such as biological extracts, for example, placental extracts, collagen, polyvitamin mixtures, and essential fatty acids.

EP-A-0189182 and EP-A-0190736 discloses GHL-Cu derivatives for enhancement of wound-healing. WO88/08695 discloses GHL-Cu derivatives for increasing hair growth and density and the subcutaneous fat content in warm-blooded animals. It refers also to general cosmetic applications WO88/08851 discloses GHL-Cu derivatives for inducing biological wound coverings in warm-blooded animals.

There exists a need in the art for improved compositions for making skin healthier, from both a structural and appearance standpoint. The present invention fulfils this need, while further providing other related advantages.

### Disclosure of the Invention

Briefly stated, the present invention discloses skin treatment and cosmetic compositions and the use thereof for maintaining and improving the health of skin. In accordance with a first aspect the present invention generally discloses the use of a composition comprising a derivative of GHL-Cu having the general formula:

(glycyl-L-histidyl-L-lysine-R): copper(II),

wherein R is selected from the group consisting of alkyl moieties containing from one to eighteen carbon atoms, aryl moieties containing from six to twelve carbon atoms, alkoxy moieties containing from one to eighteen carbon atoms, and aryloxy moieties containing from six to twelve carbon atoms, or where R is L-propyl-L-valyl-L-phenylalanyl-L-valine or L-valyl-L-phenylalanyl-L-valine, for the manufacture of a medicament for increasing the thickness of dermis, epidermis and subcutis component of the skin, the dermal density of the skin and the cell turnover rate in human skin. Within a preferred embodiment, the carbon portion of the alkoxy moiety is an unbranched chain, such as an n-octyl moiety. Further, the carbon portion of the alkoxy moiety may be an n-stearyl moiety or an n-palmityl moiety.

The disclosed compositions by virtue of their skin health promoting characteristics, also have a marked cosmetic effect, leaving the skin with a soft, pleasing, fresh appearance.

In accordance with a second aspect the present invention discloses a skin treatment composition comprising glycyl-(3-methyl)-L-histidyl-L-lysine: copper (II) and the use thereof. In addition to the derivatives described above, other chemical modifications may be made to alter the biological activity of the disclosed derivatives. For instance, the histidyl residue may be modified by the substitution of N^{tau}-methyl-histidine or (3-methyl)-histidine. Moreover, glycine may be replaced by a variety of other small amino acids, including alanine, serine and valine. Further, the copper (II) binding affinity of the molecule may be increased by addition of an N-terminal amino acid such as glycine to convert glycyl-L-histidyl-L-lysine to glycyl-L-glycyl-L-histidyl-L-lysine. In addition, glycine may be added to a derivative as described above to create the corresponding tetrapeptide.

The compositions described herein may be injected intradermally or applied topically, and are rendered suitable for administration to warm-blooded animals for the purposes of the present invention by combining the derivative with a vehicle which adapts the composition for either intradermal injection or topical application. Suitable topical formulations may be prepared with common cosmetic, nontoxic, nonallergenic carriers for use in skin creams, lotions, sprays, liquids, emollients, cleansing preparations and the like.

Other aspects of the present invention will become evident upon reference to the following detailed description and attached drawings.

### Brief Description of the Drawings

Figure 1 is a microphotograph of a biopsy section illustrating the formation of a heavy field of large, subcutaneous fat cells, as shown toward the right side of the figure.

Figure 2 is a microphotograph of a biopsy section illustrating the formation of a subcutaneous fat cell layer in the area near the injection site.

Figure 3 is a microphotograph of a control area (bottom) and an area of increased subcutaneous fat cells (top) generated through use of a representative derivative of the present invention.

Figure 4 illustrates increased alkaline phosphatase activity of biopsies taken from an animal treated with a representative derivative of the present invention.

### Best Mode for Carrying Out the Invention

As briefly noted above, skin characteristics change as humans age, and the skin's ability to both resist insults and restore itself diminishes. Skin loses its suppleness and softness, becomes thinner due to less collagen and subcutaneous fat in the skin, attains a rougher surface, is often populated by areas of damaged skin ("aging spots"), and is more wrinkled. Table 1 illustrates the histologic features of aging human skin:

**TABLE 1**

| HISTOLOGIC FEATURES OF AGING HUMAN SKIN | | |
|---|---|---|
| Epidermis | Dermis | Appendages |
| Flattened dermoepidermal junction | Atrophy (loss of dermal volume) | Depigmented hair |
| Variable thickness | Fewer fibroblasts | Loss of hair |
| Variable cell size and shape | Abnormal nerve endings | Abnormal nailplates |
| Occasional nuclear atypia | Fewer mast cells | Fewer glands |
| Fewer Langerhans cells | Fewer blood vessels | Conversion of terminal to vellus hair |

As described herein, GHL-Cu and various derivatives of GHL-Cu may be used to improve the health of skin in individuals and other warm-blooded animals. In addition, these derivatives can be tailored to increase their fat solubility, resulting in a form of the molecule which is more useful in a formulation of pharmaceutical and cosmetic creams and gels.

The disclosed compositions function to improve skin health by acting, in part, as potent in vivo chemoattractants for cells important in the maintenance of skin defenses and health, such as macrophages, monocytes and mast cells. These white cells both protect the skin from invading organisms and secrete growth factors, such as epidermal growth factor, fibroblast growth factor, platelet-derived growth factor and transforming growth factor, that function in the maintenance of healthy skin cells.

The disclosed compositions are also angiogenic and can induce new capillary growth into elderly skin that lacks sufficient blood flow. Much of the attractive appearance of the skin of young children is due to the heavy blood flow through capillaries near the skin surface. This imparts a reddish component to the skin color which increases attractiveness to the skin. As humans age, this reddish component is reduced, giving a more colorless skin.

The disclosed compositions also have significant superoxide dismutase-like activity, a property linked with anti-inflammatory effects which act by detoxifying skin-damaging oxygen radicals.

The disclosed compositions also stimulate the production of the major skin protein, collagen, by fibroblasts. Much of the wrinkling in older skin is due to a reduction in the collagen content of the skin.

The disclosed derivatives are described in detail in EP Patent Publication Nos. 288,278 and 190,736, and U.S. Patent No. 4,665,054, The disclosed derivatives may be prepared by esterification, by the removal of a water molecule, or by the addition of a group (either an alcohol such as octanol, methanol, benzol alcohol or NH₃) to the carboxylic acid terminus of GHL, resulting in the formation of the more lipophilic derivative. This increases fat solubility by (1) removal of the electric charge associated with the carboxylic acid group and (2) the introduction of hydrophilic groups into the molecule.

The chemical reaction in this transformation may be characterized as:

GHL - OH + R - H ---> GHL - R + H₂O.

In practice, the reaction is most readily carried out by adding the R group to the amino acid lysine prior to the combination of lysine with the other two amino acids to GHL. After the formation and isolation of GHL-R, the copper (II) is chelated to the molecule to form the bioactive complex.

The overall reaction to form the more lipophilic derivatives of GHL-Cu may be characterized:
1) lysine-OH + RH -----> lysine-R + H₂O
2) lysine-R + blocked L-histidine -----> blocked L-histidine-L-lysine-R
3) blocked L-histidine-L-lysine-R + blocked glycine -----> blocked glycyl-L-histidine-L- lysine-R
4) blocked glycyl-L-histidine-L-lysine-R -----> glycyl-L-histidine-L-lysine-R
5) glycyl-L-histidine-L-lysine-R + copper(II) ----> glycyl-L-histidine-L-lysine-R: copper(II).

Within preferred embodiments, the derivative of GHL and copper are present in a 1:1 or 2:1 ratio.

The derivatives of the present invention have clinical use in at least three primary areas:
(1) improving and/or maintaining the health of skin,
(2) increasing the subcutaneous fat content, and (3) in general cosmetic applications. These cosmetic applications include: (a) improving skin softness and suppleness, (b) increasing skin depth and reducing wrinkles, (c) reducing aging spots, (d) reducing skin nodules and pimples, and (e) clearing microhemorrhages and petechiae from the skin surface.

It is generally preferred to administer the derivatives described herein intradermally or topically in the center of the area to be treated, along with a suitable vehicle in a concentration of approximately 50 micrograms of derivative per 0.1 ml of vehicle. It is preferable to use a dosage of approximately 9 micrograms per cm² of area to be treated, although dosages greater than 9 micrograms/cm², up to approximately 40 micrograms/cm², may be used. Suitable vehicles in this regard include saline. When used in the form of a cream or gel and applied topically, it is useful to add a suitable penetrating agent, such as DMSO (U.S. Patent No. 3,527,864) or eucalyptol (U.S. Patent No. 4,560,553), to the composition. Suitable vehicles for use in cosmetic applications will be evident to those skilled in the art.

For topical application, the compositions of the present invention may be in the form of a cream, gel, milk, lotion or oil for the skin. Further, the compositions may be coupled with suitable excipients adapted for application to the face and the neck. Appropriate excipients should have a high affinity for the skin, be well tolerated, stable, and present an adequate consistency enabling easy and pleasant utilization. Examples of excipients in this regard include a mixture of isopropyl myristate, gylcerol sterate, sweet almond oil and polyhydric alcohol (respectively 5 grams (g) - 15 g - 6 g - 5 g for 100 ml of distilled water).

Additional ingredients may be added according to the understanding of those familiar with the art in order to vary the texture, consistency, viscosity, and appearance of the formulation. These additional ingredients include emulsifying agents such as nonionic ethoxylated and nonethexylated surfactants, fatty alcohols, fatty acids, organic or inorganic bases, preserving agents, wax esters, steroid alcohols, triglyceride esters, phospholipids such as lecithin and cephalin, polyhydric alcohol esters, fatty alcohol ethers, hydrophilic lanolin derivatives, hydrophilic beeswax derivatives, hydrocarbon oils such as palm oil, coconut oil, mineral oil, cocoa butter waxes, silicon oils, pH balancers and cellulose derivatives.

The disclosed compositions may also contain small quantities of solar radiation filters or sunscreens, for example, UV-A and UV-B radiation filters, such as hydroxy 2-methoxy 4-benzophene, and dimethoxy 3,4-phenyl glyoxylic acid in the form of its sodium salt. The compositions may further contain humectants favoring the hydration of the skin such as urea, pyrrolidone carboxilic acid and its salts, vitamin extracts, perfumes, preservatives and colors.

To summarize the examples which follow, Example I illustrates the synthesis of glycyl-L-histidyl-L-lysine benzyl ester:copper(II). Example II demonstrates the synthesis of glycyl-L-histidyl-L-lysine n-octyl ester:copper(II). Example III illustrates (A) the synthesis of glycyl-L-histidyl-L-lysine n-stearyl ester:copper(II), and (B) its synthesis by an alternative procedure. Based upon either procedure, one skilled in the art could substitute n-palmityl alcohol (16 carbons) for the n-stearyl alcohol (18 carbons) to yield glycyl-L-histidyl-L-lysine n-stearyl ester: copper(II). Example IV illustrates the synthesis of glycyl-L-histidyl-L-lysyl-L-prolyl-L-valyl-L-phenylalanyl-L-valine:copper(II) and glycyl-L-histidyl-L-lysyl-L-valyl-L-phenylalanyl-L-valine:copper(II). Examples V, VI, and VII demonstrate the use of various derivatives of the present invention to stimulate or increase the formation of the subcutaneous fat layer. Example VIII demonstrates the use of a representative composition of the present invention in clearing microhemorrhages from the skin surface. Example IX demonstrates the use of a representative composition of the present invention in reducing skin nodules. Example X demonstrates the use of a representative composition of the present invention in reducing the depth of wrinkles. Example XI demonstrates the use of a representative composition of the present invention in treating skin characterized by an eczema-like surface. Example XII demonstrates the use of a representative composition of the present invention in reducing aging spots. Example XIII demonstrates the use of a representative composition of the present invention in the treatment of pimples. Example XIV illustrates the synthesis of glycyl-N^{tau}-methyl-L-histidyl-L-lysine. Example XV demonstrates the use of glycyl-(3-methyl)-L-histidyl-L-lysine:copper(II) to stimulate angiogenesis and collogen synthesis in young pigs. Example XVI demonstrates the use of glycyl-L-histidyl-L-lysine:copper(II), glycyl-(3-methyl)-L-histidyl-L-lysine:copper(II), and glycyl-L-histidyl-L-lysyl-L-valyl-L-phenylanlanyl-L-valine:copper(II) to increase the thickness of the dermis, epidermis and subcutis components in a warm-blooded animal. Example XVII illustrates the changes in papillary and reticular dermis in subjects treated with a cream containing glycyl-(3-methyl)-L-histidyl-L-lysine:copper(II). Example XVIII illustrates the increased cell turnover rate in human epidermis treated with a cream containing glycyl-(3-methyl)-L-histidyl-L-lysine:copper(II).

The following examples are offered by way of illustration.

### EXAMPLES

Sources of chemicals. Chemicals and peptide intermediates utilized in the following examples may be purchased from the following suppliers: Sigma Chemical Co. (St. Louis, Mo.); Peninsula Laboratories (San Carlos, Calif.); Aldridge Chemical Co. (Milwaukee, Wis.); Vega Biochemicals (Tucson, Ariz.); Pierce Chemical Co. (Rock ford, Ill.); Research Biochemicals (Cleveland, Ohio); Van Waters and Rogers (South San Francisco, Calif.); Bachem, Inc. (Torrance, Calif.).

### EXAMPLE I

### Synthesis of glycyl-L-histidyl-L-lysine benzyl ester: copper(II)

N^{e}-benzyloxycarbonyl-L-lysine benzyl ester was dissolved in 1:1 hexane-ethyl acetate and coupled to N^{a}-t-butyloxycarbonyl-N^{im}-benzyloxycarbonyl-L-histidine using dicyclohexylcarbodiimide as a coupling agent. Sodium bicarbonate (10%) was added and the product extracted into the organic layer. The product, N^{a}-t-butyloxycarbonyl-N^{im}-benzyloxycarbonyl-L-histidyl-N^{e}-benzyloxycarbonyl-L-lysine benzyl ester, was crystallised from solution. The N-terminal group of the blocked dipeptide was removed by stirring in 50% trifluoroacetic acid in dichloromethane for 30 minutes, then vacuum evaporated. The product, N^{im}-benzyloxycarbonyl-L-histidyl-N^{e}-benzoylcarbonyl-L-lysine benzyl ester, was coupled to benzyloxycarbonyl-glycine with dicyclohexylcarbodiimide as a coupling agent. Blocking groups were removed by catalytic hydrogenation using 10% palladium on carbon in glacial acetic acid. After lyophilization, the product, glycyl-L-histidyl-L-lysine benzyl ester, was dissolved in water and purified by ion-exchange chromatography on Dowex 50 X4 cation-exchange resin and elution with 0.1 M ammonium hydroxide, the eluate being immediately neutralized with acetic acid. A further passage through an anionexchange column BioRex 63 at neutral pH removed breakdown products with free carboxylic acid groups.

The glycyl-L-histidyl-L-lysine benzyl ester was dissolved in water with equimolar copper acetate added. The pH was raised to neutrality with sodium hydroxide. The solution was centrifuged at 20,000 x ̂g for 1 hour at 3°C to remove poorly water soluble material. The supernatant was lyophilized to obtain glycyl-L-histidyl-L-lysine benzyl ester:copper(II).

### EXAMPLE II

### Synthesis of glycyl-L-histidyl-L-lysine n-octyl ester: copper (II)

A mixture of N^{e}-benzyloxycarbonyl-L-lysine, n-octanol, benzene, and p-toluenesulfonic acid monohydrate was refluxed overnight using a Dean-Stark trap to remove water. After cooling, dry ethyl ether was added. The solution was then allowed to precipitate at 0°C overnight. A portion of the precipitated solid was added to 50 ml potassium carbonate solution and 50 ml dichloromethane. After extraction, the layers were separated and the organic phase washed with water and brine, then dried with anhydrous magnesium sulfate. Filtration, evaporation and purification by flash column chromatography gave n-octyl N^{e}-benzyloxycarbonyl-L-lysinate. The product was dissolved in tetrahydrofuran and mixed with N^{a}-t-butyloxycarbonyl-L-N^{im}-benzyloxycarbonyl-L-histidine, isobutyl chloroformate and N-methylmorpholine. After evaporation, water and ethyl acetate were added. The product was extracted into the organic phase, which was dried with anhydrous magnesium sulfate. Filtration, evaporation and purification by flash column chromatography gave n-octyl N^{a}-t-butyloxycarbonyl N^{im}-benzyloxycarbonyl-L-histidyl-N^{e}-benzyloxycarbonyl-L-lysinate.

The product was dissolved in 50% trifluoroacetic acid in dichloromethane for 30 minutes, then evaporated, forming n-octyl N^{im}-benzyloxycarbonyl-L-histidyl-N^{e}-benzyloxycarbonyl-L-lysinate. This was dissolved in tetrahydrofuran, and isobutyl chloroformate, N-methylmorpholine and benzyloxycarbonylglycine were added to form n-octyl benzyloxycarbonylglycyl-N^{im}-benzyloxycarbonyl-L-histidyl-N^{e}-benzyloxycarbonyl-L-lysinate. This was dissolved in glacial acetic acid and hydrogenated overnight.

The resultant n-octyl ester of glycyl-L-histidyl-L-lysine was converted to the copper complex by the addition of an equimolar quantity of copper diacetate. The pH was raised to neutrality with sodium hydroxide. The solution was centrifuged at 20,000 x ̂g for 1 hour at 3°C to remove poorly water-soluble material. The supernatant was lyophilized to obtain glycyl-L-histidyl-L-lysine n-octyl ester:copper(II).

### EXAMPLE III

### A. Synthesis of glycyl-L-histidyl-L-lysine n-stearyl ester:copper(II)

A mixture of N^{e}-benzyloxycarbonyl-L-lysine, n-stearyl alcohol, benzene, and p-toluenesulfonic acid monohydrate was refluxed overnight using a Dean-Stark trap to remove water. After cooling, dry propyl ether was added to increase the total volume sixfold. The product was allowed to precipitate at 0°C overnight and filtered. A portion of the filtrate was added to 50 ml potassium carbonate and 50 ml dichloromethane. After extraction, the layers were separated, and the organic phase was washed with water and brine, then dried with anhydrous magnesium sulfate. Filtration, evaporation and purification by flash column chromatography gave n-stearyl N^{e}-benzyloxycarbonyl-L-lysinate. The product was dissolved in tetrahydrofuran and mixed with N^{a}-t-butyloxycarbonyl-N^{im}-benzyloxycarbonyl-L-histidine and isobutyl chloroformate and N-methylmorpholine. After evaporation, water and propyl acetate were added and the product was extracted into the organic phase, then dried with anhydrous magnesium sulfate. Filtration, evaporation and purification by flash column chromatography gave n-stearyl N^{a}-t-butyloxycarbonyl-N^{im}-benzyloxycarbonyl-L-histidyl-N^{e}-benzyloxycarbonyl-L-lysinate.

The product was dissolved in 50% trifluoroacetic acid in dichloromethane for 30 minutes, then evaporated, forming n-stearyl N^{im}-benzyloxycarbonyl-L-histidyl-N^{e}-benzyloxycarbonyl-L-lysinate, which was dissolved in tetrahydrofuran, isobutyl chloroformate, N-methylmorpholine and benzyloxycarbonylglycine to form n-stearyl benzyloxycarbonylglycyl-N^{im}-benzyloxycarbonyl-L-histidyl-N^{e}-benzyloxycarbonyl-L-lysinate. The product was dissolved in 50% trifluoroacetic acid in dichloromethane for 30 minutes, then evaporated, forming n-stearyl ester glycyl-L-histidyl-L-lysine.

The resultant molecule, glycyl-L-histidyl-L-lysine n-stearyl ester, was converted to the copper complex by the addition of an equimolar quantity of copper diacetate. The pH was raised to neutrality with sodium hydroxide to obtain a product useful for animal studies.

By substituting n-palmityl alcohol for the n-stearyl alcohol, glycyl-L-histidyl-L-lysine n-palmityl ester may be similarly synthesized.

### B. Alternative synthesis of glycyl-L-histidyl-L-lysine n-stearyl ester:copper(II)

N(ε)-benzyloxycarbonyl-L-lysine, n-stearyl alcohol, p-toluenesulfonic acid monohydrate, and benzene are refluxed together using a Dean-Stark trap to azeotropically remove the evolved water. After cooling to room temperature and then adding dry ethyl ether, n-stearyl N(ε)-benzyloxycarbonyl-L-lysinate p-toluenesulfonate salt is collected by filtration, treated with 2 ̂M aqueous potassium bicarbonate solution, and extracted into dichloromethane. Evaporation gives the free amine, which is redissolved in dry tetrahydrofuran (THF) and added to a stirring solution of N(α)-t-butyloxycarbonyl-N(im)-benzyloxycarbonyl-L-histidine, N-methylmorpholine, and isobutyl chloroformate in dry THF at -15°C. The resulting fully protected dipeptide ester is treated with 1/1 trifluoroacetic acid/ dichloromethane at room temperature, neutralized with saturated agueous sodium bicarbonate solution, and extracted into ethyl acetate. Evaporation gives the partially deblocked dipeptide, which is redissolved in dry THF and added to a stirring solution of benzyloxycarbonylglycine, N-methylmorpholine and isobutyl chloroformate in dry THF at -15°C. The formed, fully protected tripeptide ester is totally deblocked by treatment with hydrogen gas in glacial acetic acid at room temperature in the presence of Pd-C catalyst. Filtration, evaporation and purification on a microcrystalline cellulose column followed by lyophilization give the desired tripeptide ester as its triacetate salt.

The resultant molecule, glycyl-L-histidyl-L-lysine n-stearyl ester, was converted to the copper complex by the addition of an equimolar quantity of copper diacetate. The pH was raised to neutrality with sodium hydroxide to obtain a product useful for animal studies.

By substituting n-palmityl alcohol for the n-stearyl alcohol, glycyl-L-histidyl-L-lysine n-palmityl ester may be similarly synthesized.

### EXAMPLE IV

### Synthesis of glycyl-L-histidyl-L-lysyl-L-prolyl-L-valyl-L-phenylalanyl-L-valine:copper(II) and of glycyl-L-histidyl-L-lysyl-L-valyl-L-phenylalanyl-L-valine: copper(II)

These peptides are synthesized by standard solid-phase methods common to the peptide field (J. Stewart and J. Young, Solid Phase Peptide Synthesis, Pierce Chemical Co., 1984). Briefly stated, Boc-Val-O-Resin was sequentially coupled with other blocked amino acids using dicyclohexylcarbodiimide as a reaction agent. Protected amino acids, resins for solid-phase synthesis, and coupling agents were obtained from Peninsula Laboratories, San Carlos, California. Blocked amino acids are added in sequential order to obtain the desired peptide. The final peptide is deblocked using hydrogen fluoride. The final peptide is dissolved in 0.5% acetic acid and purified by passage through a Sephadex G-15 column (Pharmacia). Addition of equimolar cupric acetate, followed by lyophilization, produces the active molecule.

### EXAMPLE V

### Use of glycyl-L-histidyl-L-lysine n-octyl ester: copper(II) to increase the formation of the subcutaneous fat layer

A single dose of 50 micrograms of glycyl-L-histidyl-L-lysine n-octyl ester:Cu(II) was infiltrated under the skin in eight mice. Increased amounts of subcutaneous fat were observed.

In another series of experiments, mice were injected once with glycyl-L-histidyl-L-lysine octyl ester:copper(II) at a dose of 500 micrograms per mouse.

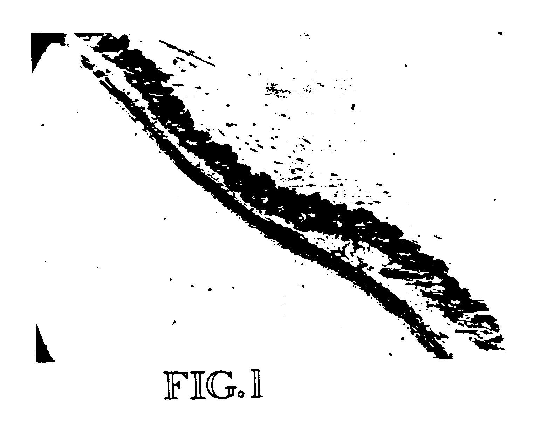

In the region of administration, there was a significant increase in the thickness of the subcutaneous fat layer. This increase in subcutaneous fat is shown by taking a biopsy sample at day 21 through the area and sectioning for histology slides. Figure 1 shows this increase in the fat layer. The injected area is on the right with the adjacent uninjected area on the left of the photograph. There was an increase in both the number and size of the fat cells. Measurements demonstrate that there was an approximately threefold increase in the subcutaneous fat layer in the skin near the injection site.

### EXAMPLE VI

### Use of glycyl-L-histidyl-L-lysine decyl ester:copper(II) to stimulate formation of the subcutaneous fat layer

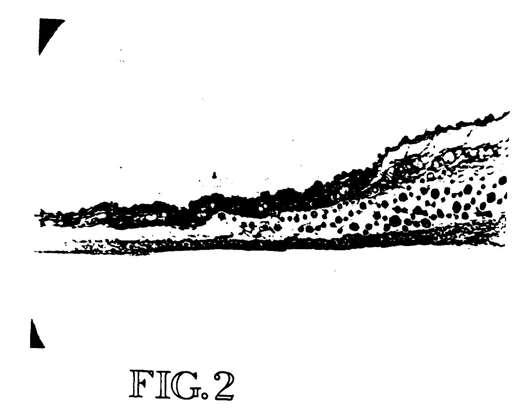

A group of ten mice were injected once with glycyl-L-histidyl-L-lysine decyl ester:copper(II) at a dose of 500 micrograms per mouse. Microscopic examination provided evidence of increased fat cell layer in the area surrounding the injection site. Figure 2 shows this marked increase in the subcutaneous fat layer at the area of injection. Examination of the skin distant from the injection site showed a normal histology.

### EXAMPLE VII

### Use of glycyl-L-histidyl-L-lysine palmityl ester: copper(II) to increase the formation of the subcutaneous fat layer

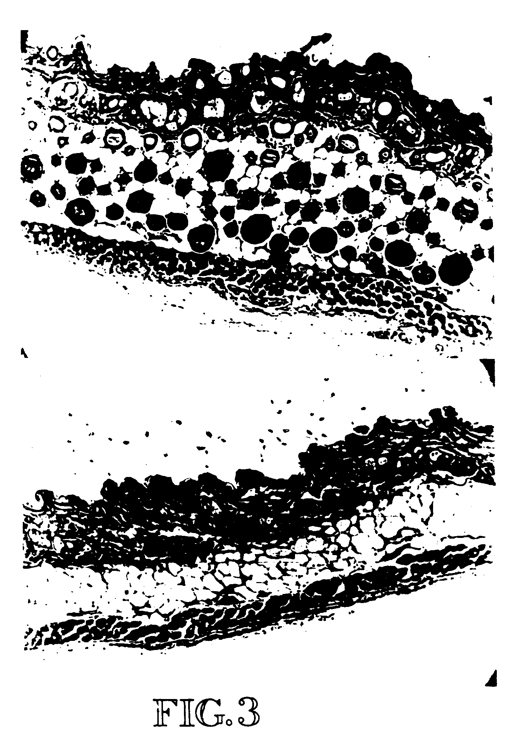

A group of ten mice were injected once with glycyl-L-histidyl-L-lysine palmityl ester:copper(II) at a dose of 500 micrograms per mouse. Histological sections through the area of injection were similar to those described in Example VI. Figure 3 is a photomicrograph demonstrating an increase in the subcutaneous fat layer, similar to that seen following the glycyl-L-histidyl-L-lysine decyl ester:Cu injection.

### EXAMPLE VIII

An 82-year-old woman had skin covered by a sprinkled pattern of fine reddish spots of microhemorrhages beneath an irritated skin surface. Application, once daily for 9 days, of an ointment (97% Unibase and 3% nonoxynol-9) containing 4 mg GHL-Cu per gram resulted in a complete clearing of the reddish spots and an improved skin appearance.

### EXAMPLE IX

A 73-year-old woman had a skin surface with numerous small reddish elevated nodules (slightly smaller than pimples). Treatment with the ointment used in Example VIII resulted in a marked reduction in the nodules within 10 days. By 20 days, the skin was completely clear with a smooth, healthy, and attractive appearance.

### EXAMPLE X

In a 72-year-old woman who had skin covered with heavy wrinkles, application of the cream used in Example VIII once daily for the first five days, and every other day for 23 days markedly reduced the depth of wrinkles and gave a smoother skin appearance.

### EXAMPLE XI

A 77-year-old woman had skin covered with a scaly eczema-like surface. Treatment of the skin once daily for the first five days, and every other day for 40 days with the ointment used in Example VIII resulted in a sloughing off of the scaly skin and its replacement by a new and more attractive layer of skin.

### EXAMPLE XII

A 48-year-old man with hair loss and numerous pigmented spots ("aging spots") was treated for 14 days on the top of his head with an ointment (consisting of 94% Unibase and 6% dimethysulfoxide) containing 4 milligrams of GHL-Cu octyl per gram. Thirty days after the start of the treatment, there was a marked reduction in the number of aging spots and a reduction in the size of the remaining spots. The skin covering the head became noticeably softer to the touch and possessed a greater thickness.

### EXAMPLE XIII

A 40-year-old woman had irritated skin with numerous pimples. Treatment once daily for four days with the ointment used in Example VIII resulted in a clearing of the skin and a reduction in the pimple size. By eight days after the start of the treatment, the pimples were completely resorbed, leaving a clear and attractive skin surface.

### EXAMPLE XIV

### Synthesis of glycyl-N^{tau}-methyl-L-histidyl-L-lysine

N^{e}-benzyloxycarbonyl-L-lysine benzyl ester hydrochloride salt was suspended in tetrahydrofuran (THF) and neutralized with one equivalent of N-methylmorpholine. It was then coupled with N^{a}-t-butyloxycarbonyl-N^{tau}-methyl-L-histidine using isobutyl chloroformate and N-methylmorpholine in THF. After two hours at -20°C and an additional hour at ambient temperature, the reaction was quenched with 2 N aqueous potassium bicarbonate. The product was extracted into ethyl acetate, washed with 1 M aqueous citric acid, and saturated sodium bicarbonate. The organic phase was dried over anhydrous sodium sulfate. Filtration and evaporation gave benzyl N^{a}-t-butyloxycarbonyl-N^{tau}-methyl-L-histidyl-N^{e}-benzyloxycarbonyl-L-lysinate.

The product was dissolved in 30% trifluoroaceteic acid in dichloromethane for 30 minutes, then evaporated, forming benzyl N^{tau}-methyl-L-histidyl-N^{e}-benzyloxycarbonyl-L-lysinate. This was dissolved in tetrahydrofuran; and isobutyl chloroformate, N-methylmorpholine and benzyloxycarbonylglycine were added to form benzyl benzyloxycarbonylglycyl-N^{tau}-methyl-L-histidyl-N^{e}-benzyloxycarbonyl-L-lysinate. This product was then dissolved in acetic acid and hydrogenated overnight in the presence of 10% Pd-C catalyst. The resultant glycyl-N^{tau}-methyl-L-histidyl-L-lysine was lyophilized from water several times, then purified by liquid chromatography on a C-18 reverse-phase column to yield the desired tripeptide as a diacetate salt.

### EXAMPLE XV

### Use of glycyl-(3-methyl)-L-histidyl-L-lysine:copper(II) to stimulate angiogenesis and collagen synthesis in young pigs

In a typical experiment, a young pig was treated topically with a cream containing glycyl-(3-methyl)-L-histidyl-L-lysine:copper(II) or a placebo cream once a day for 12 days. Punch biopsies (6 mm dia.) were taken on day 0 and day 12. The biopsies were analyzed for total wet weight, alkaline phosphatase, total protein, and hydroxyproline content. Alkaline phosphatase is an enzyme marker for capillary endothelial cells and is an indicator of new angiogenesis. The hydroxyproline is a component of collagen, and increased hydroxyproline content indicates increased collagen content. The punch biopsies were prepared for biochemical analysis, basically by the method of Counts et al. (D. Counts, P. Knighten and G. Hegreberg, J. Invest. Derm. 69:521-26, 1977).

In a first experiment, an increase in alkaline phosphatase activity and hydroxyproline content was detected on day 12 in the biopsies taken from skin treated with glycyl-(3-methyl)-L-histidyl-L-lysine:copper(II) relative to the placebo-treated area. The results of this experiment are presented in Table 2.

**TABLE 2**

| COLLAGEN CONTENT AND ANGIOGENIC ACTIVITY OF PIG SKIN BIOPSIES | | |
|---|---|---|
| | Collagen Content (as hydroxyproline) (µg/biopsy) | Angiogenic Activity (as alkaline phosphatase) (inc A405/min/mg) |
| CONTROL | 3.2 ± 0.2 | 0.010 ± 0.001 |
| TREATED | 5.8 ± 0.2 (+ 80%) | 0.013 ± 0.001 (+ 30%) |

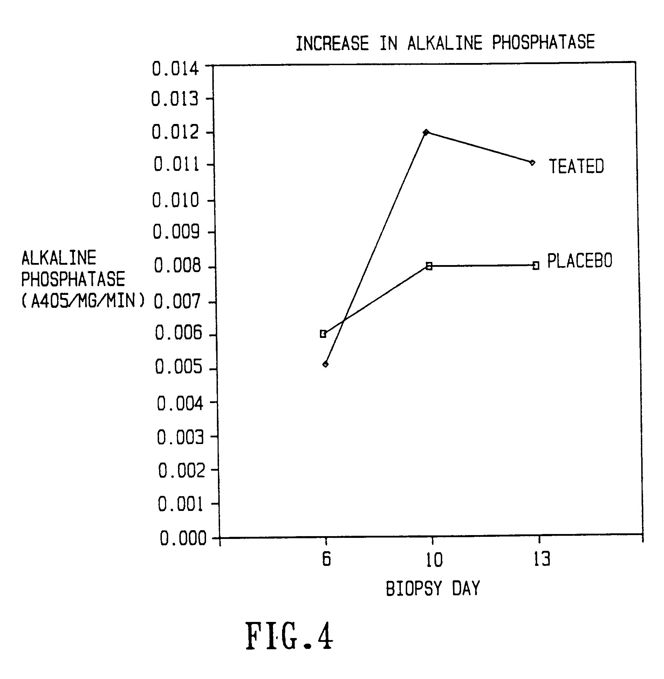

The results obtained in a second experiment are graphically illustrated in Figure 4. In this experiment, biopsies were taken from the pig on days 0, 10 and 13 and analyzed for alkaline phosphatase activity. The biopsies taken from areas treated with glycyl-(3-methyl)-L-histidyl-L-lysine:copper(II) had increased alkaline phosphatase relative to the placebo cream-treated areas.

### EXAMPLE XVI

### Use of glycyl-L-histidyl-L-lysine:copper(II). glycyl-(3-methyl)-L-histidyl-L-lysine:copper(II), and glycyl-L-histidyl-L-lysyl-L-valyl-L-phenylalanyl-L-valine: copper(II) to increase the thickness of dermis. epidermis and subcutis components in mice

Skin from 31 Swiss-Webster female mice (average age - 18 months) was evaluated histologically for evidence of changes in skin architecture following several applications of placebo- and peptide-containing cream formulations. Measurements were made of dermal, epidermal and subcutis thickness using an eyepiece micrometer at 100X power.

The mice had hair removed by a close clipping, and were treated on days 0, 1, 3, 4 and 5 with either glycyl-L-histidyl-L-lysine:copper(II), or representative derivatives glycyl-(3-methyl)-L-histidyl-L-lysine: copper(II) and glycyl-L-histidyl-L-lysyl-L-valyl-L-phenylalanyl-L-valine: copper(II), or a placebo cream (n = 5/group). Biopsies were performed on individual animals from the groups on days 6, 10, 12 and 14. In addition, eleven naive mice were biopsied on day 0.

Treatment with cream formulations caused a measurable increase in the thickness of the dermis, epidermis and subcutis components. On day 6, the epidermal layer had increased on all cream-treated mice (including placebo-cream treated mice) from approximately 13 microns to approximately 42 microns. By day 14, the effect of increased epidermal thickness had reversed in the placebo and was approximately the same as the naive controls. Mice treated with either glycyl-L-histidyl-L-lysyl-L-valyl-L-phenylalanyl-L-valine: copper(II)- or glycyl-(3-methyl)-L-histidyl-L-lysine:copper(II)containing creams, however, maintained an increased epidermal thickness.

### EXAMPLE XVII

### Changes in papillary and reticular dermis in subjects treated with a cream containing glycyl-(3-methyl)-L-histidyl-L-lysine:copper(II)

The effect of a representative composition, glycyl-(-3-methyl-L-histidyl-L-lysine:copper(II)(active agent), on the epidermis and dermis was evaluated by ultrasound. Ultrasound technique uses high-frequency sound, at 20 megahertz, to produce an echo signal of the skin. This signal is processed to produce a diagrammatic representation of the skin that is related to the structures of the skin by the strength of the reflected signal.

The topical cream containing glycyl-(3-methyl)-L-histidyl-L-lysine:copper(II) or a control cream was applied twice daily for three weeks to the volar surface of the forearms of 10 female subjects with an average age of 31.7 years, using both the right and left sides in a random fashion for each cream. Subjects were evaluated by ultrasound scans at days 1, 7, 14 and 21.

The dermal density after treatment as measured by ultrasound was evaluated on a scoring system whereby no change = 0, a slight change = 1, a moderate change = 2, and a marked change = 3. In 10 women treated with the cream-plus-active-agent, the average density score was 1.7, while the average score of the placebo-cream-only was 0.7. This difference was significant at a probability of p = 0.029.

### EXAMPLE XVIII

### Increased cell turnover rate in human epidermis treated with a cream containing glycyl-(3-methyl)-L-histidyl-L-lysine:copper(II)

The rate of cell turnover is determined by the number of days for clearance of dansyl chloride from stained stratum. Seven female subjects were used in the study, with an average age of 50.4 (± 3.8) years. Each subject was treated with 3% dansyl chloride in petrolatum on four sites on the upper inner arms. One site on each arm served as an untreated control and the other site as the treated site. Sites were randomized by computer as to left or right and as to proximal or distal areas of the upper arm. Twenty-four hours after occlusion, the sites were uncovered and photographed under ultraviolet light for fluorescence of the dansyl chloride. This photograph was designated as day 1. Photographs were made over the next four weeks until all subjects had completed the study, as determined by the final disappearance of the dansyl dye. The sites were then treated daily with a topical cream containing a representative composition, glycyl-(3-methyl)-L-histidyl-L-lysine:copper(II) or a control cream. The number of days for the total disappearance of the dansyl dye from the cream-treated sites was compared with the corresponding untreated site.

The average skin turnover rate increased 30.0% in the skin sites treated with a topical cream containing glycyl-(3-methyl)-L-histidyl-L-lysine:copper(II). In contrast, the placebo cream increased turnover only 17.7% in the skin sites. This difference indicates that there was an increase in the rate of cell turnover by treatment with glycyl-(3-methyl)-L-histidyl-L-lysine: copper(II).

## Claims

1. A skin treatment composition, comprising a derivative of GHL-Cu having the general formula:
(glycyl-(3-methyl)-L-histidyl-L-lysine-R):copper(II)
wherein R is selected from the group consisting of alkyl moieties containing from 1 to 18 carbon atoms, aryl moieties containing from 6 to 12 carbon atoms, alkoxy moieties containing from 1 to 18 carbon atoms, aryloxy moieties containing from 6 to 12 carbon atoms, or where R is L-prolyl-L-valyl-L-phenylalanyl-L-valine or L-valyl-L-phenylalanyl-L-valine, in combination with a cosmetically and dermatologically acceptable carrier or diluent.

2. A skin treatment composition, comprising glycyl(3-methyl)-L-histidyl-L-lysine:copper(II), in combination with a cosmetically and dermatologically acceptable carrier or diluent.

3. A composition comprising glycyl-(3-methyl)-L-histidyl-L-lysine:copper(II), for use within a method for increasing subcutaneous fat in warm-blooded animals.

4. A composition comprising glycyl-(3-methyl)-L-histidyl-L-lysine:copper(II) for use within a method for increasing the thickness of dermis, epidermis and subcutis components of skin.

5. A composition comprising glycyl-(3-methyl)-L-histidyl-L-lysine:copper(II) for use within a method for increasing dermal density of skin.

6. A composition comprising glycyl-(3-methyl)-L-histidyl-L-lysine:copper(II) for use within a method for increasing the cell turnover rate in human epidermis.

7. A method of cosmetic treatment of human skin which method comprises the application to skin of a composition comprising glycyl-(3-methyl)-L-histidyl-L-lysine:copper(II).

8. Use of a composition comprising glycyl-(3-methyl)-L-histidyl-L-lysine:copper(II), for the manufacture of a medicament for increasing subcutaneous fat in warm-blooded animals.

9. Use of a composition comprising a derivative of GHL-Cu having the general formula:
(glycyl-L-histidyl-L-lysine-R):copper(II)
wherein R is selected from the group consisting of alkyl moieties containing from 1 to 18 carbon atoms, aryl moieties containing from 6 to 12 carbon atoms, alkoxy moieties containing from 1 to 18 carbon atoms, and aryloxy moieties containing from 6 to 12 carbon atoms, or where R is L-prolyl-L-valyl-L-phenylalanyl-l-valine or L-valyl-L-phenylalanyl-L-valine, for the manufacture of a medicament for increasing the thickness of dermis, epidermis and subcutis components of skin.

10. Use of a composition comprising glycyl-(3-methyl)-L-histidyl-L-lysine):copper(II) for the manufacture of a medicament for increasing the thickness of dermis, epidermis and subcutis components of skin.

11. Use of a composition comprising a derivative of GHL-Cu having the general formula:
(glycyl-L-histidyl-L-lysine-R-):copper(II)
wherein R is selected from the group consisting of alkyl moieties containing from 1 to 18 carbon atoms, aryl moieties containing from 6 to 12 carbon atoms, alkoxy moieties containing from 1 to 18 carbon atoms, and aryloxy moieties containing from 6 to 12 carbon atoms, or where R is L-prolyl-L-valyl-L-phenylalanyl-L-valine or L-valyl-L-phenylalanyl-L-valine, for the manufacture of a medicament for increasing the dermal density of skin.

12. Use of a composition comprising glycyl-(3-methyl)-L-histidyl-L-lysine:copper(II) for manufacture of a medicament for increasing dermal density of skin.

13. Use of a composition comprising a derivative of GHL-Cu having a general formula:
(glycyl-L-histidyl-L-lysine-R):copper(II)
wherein R is selected from the group consisting of alkyl moieties containing from 1 to 18 carbon atoms, aryl moieties containing from 6 to 12 carbon atoms, alkody moieties containing from 1 to 18 carbon atoms, and aryloxy moieties containing from 6 to 12 carbon atoms, or where R is L-prolyl-L-valyl-L-phenylalanyl-L-valine or L-valyl-L-phenylalanyl-L-valine, for the manufacture of a medicament for increasing the cell turnover rate in human epidermis.

14. Use of a composition comprising glycyl-(3-methyl)-L-histidyl-L-lysine:copper(II) for the manufacture of a medicament for increasing the cell turnover rate in human epidermis.

## Patentansprüche

1. Eine Hautbehandlungs-Zusammensetzung, die ein Derivat von GHL-Cu mit der allgemeinen Formel umfaßt:
(Glycyl-(3-methyl)-L-histidyl-L-lysin-R):Kupfer(II),
wobei R ausgewählt ist aus der Gruppe, bestehend aus Alkyleinheiten, die von 1 bis 18 Kohlenstoffatome enthalten, Aryleinheiten, die von 6 bis 12 Kohlenstoffatome enthalten, Alkoxyeinheiten, die von 1 bis 18 Kohlenstoffatome enthalten, Aryloxyeinheiten, die von 6 bis 12 Kohlenstoffatome enthalten, oder in der R L-Prolyl-L-valyl-L-phenylalanyl-L-valin oder L-Valyl-L-phenylalanyl-L-valin ist, in Kombination mit einem kosmetisch oder dermatologisch annehmbaren Trägerstoff oder Verdünnungsmittel.

2. Eine Hautbehandlungs-Zusammensetzung, die Glycyl-(3-methyl)-L-histidyl-L-lysin:Kupfer(II) umfaßt, in Kombination mit einem kosmetisch oder dermatologisch annehmbaren Trägerstoff oder Verdünnungsmittel.

3. Eine Zusammensetzung, die Glycyl-(3-methyl)-L-histidyl-L-lysin:Kupfer(II) umfaßt, zur Verwendung in einem Verfahren zur Erhöhung von Unterhautfett bei Warmblütern.

4. Eine Zusammensetzung, die Glycyl-(3-methyl)-L-histidyl-L-lysin:Kupfer(II) enthält, zur Verwendung in einem Verfahren zur Erhöhung der Dicke von Dermis-, Epidermis- und Subcutis-Komponenten von Haut.

5. Eine Zusammensetzung, die Glycyl-(3-methyl)-L-histidyl-L-lysin:Kupfer(II) umfaßt, zur Verwendung in einem Verfahren zur Erhöhung der dermalen Dichte von Haut.

6. Eine Zusammensetzung, die Glycyl-(3-methyl)-L-histidyl-L-lysin:Kupfer(II) umfaßt, zur Verwendung in einem Verfahren zur Steigerung der Zellumsatzrate in menschlicher Epidermis.

7. Ein Verfahren zur kosmetischen Behandlung menschlicher Haut, wobei das Verfahren das Aufbringen einer Zusammensetzung, die Glycyl-(3-methyl)-L-histidyl-L-lysin: Kupfer(II) umfaßt, auf Haut umfaßt.

8. Verwendung einer Zusammensetzung, die Glycyl-(3-methyl)-L-histidyl-L-lysin:Kupfer(II) umfaßt, zur Herstellung eines Arzneimittels zur Erhöhung von Unterhautfett bei Warmblütern.

9. Verwendung einer Zusammensetzung, die ein Derivat von GHL-Cu mit der allgemeinen Formel umfaßt:
(Glycyl-L-histidyl-L-lysin-R):Kupfer(II),
wobei R ausgewählt ist aus der Gruppe, bestehend aus Alkyleinheiten, die von 1 bis 18 Kohlenstoffatome enthalten, Aryleinheiten, die von 6 bis 12 Kohlenstoffatome enthalten, Alkoxyeinheiten, die von 1 bis 18 Kohlenstoffatome enthalten, und Aryloxyeinheiten, die von 6 bis 12 Kohlenstoffatome enthalten, oder in der R L-Prolyl-L-valyl-L-phenylalanyl-L-valin oder L-Valyl-L-phenylalanyl-L-valin ist, zur Herstellung eines Arzneimittels zur Erhöhung der Dicke von Dermis-, Epidermis- und Subcutis-Komponenten von Haut.

10. Verwendung einer Zusammensetzung, die Glycyl-(3-methyl)-L-histidyl-L-lysin:Kupfer(II) umfaßt, zur Herstellung eines Arzneimittels zur Erhöhung der Dicke von Dermis-, Epidermis-und Subcutis-Komponenten von Haut.

11. Verwendung einer Zusammensetzung, die ein Derivat von GHL-Cu mit der allgemeinen Formel umfaßt:
(Glycyl-L-histidyl-L-lysin-R):Kupfer(II),
wobei R ausgewählt ist aus der Gruppe, bestehend aus Alkyleinheiten, die von 1 bis 18 Kohlenstoffatome enthalten, Aryleinheiten, die von 6 bis 12 Kohlenstoffatome enthalten, Alkoxyeinheiten, die von 1 bis 18 Kohlenstoffatome enthalten, und Aryloxyeinheiten, die von 6 bis 12 Kohlenstoffatome enthalten, oder in der R L-Prolyl-L-valyl-L-phenylalanyl-L-valin oder L-Valyl-L-phenylalanyl-L-valin, zur Herstellung eines Arzneimittels zur Erhöhung der dermalen Dichte von Haut.

12. Verwendung einer Zusammensetzung, die Glycyl-(3-methyl)-L-histidyl-L-lysin:Kupfer(II) umfaßt, zur Herstellung eines Arzneimittels zur Erhöhung der dermalen Dichte von Haut.

13. Verwendung einer Zusammensetzung, die ein Derivat von GHL-Cu mit einer allgemeinen Formel umfaßt:
(Glycyl-L-histidyl-L-lysin-R):Kupfer(II),
wobei R ausgewählt ist aus der Gruppe, bestehend aus Alkyleinheiten, die von 1 bis 18 Kohlenstoffatome enthalten, Aryleinheiten, die von 6 bis 12 Kohlenstoffatome enthalten, Alkoxyeinheiten, die von 1 bis 18 Kohlenstoffatome enthalten, und Aryloxyeinheiten, die von 6 bis 12 Kohlenstoffatome enthalten, oder in der R L-Prolyl-L-valyl-L-phenylalanyl-L-valin oder L-Valyl-L-phenylalanyl-L-valin ist, zur Herstellung eines Arzneimittels zur Erhöhung der Zellumsatzrate in menschlicher Epidermis.

14. Verwendung einer Zusammensetzung, die Glycyl-(3-methyl)-L-histidyl-L-lysin:Kupfer(II) umfaßt, zur Herstellung eines Arzneimittels zur Erhöhung der Zellumsatzrate in menschlicher Epidermis.

## Revendications

1. Composition de traitement cutané, comprenant un dérivé de GHL-Cu répondant à la formule générale :
(glycyl-(3-méthyl)-L-histidyl-L-lysine-R):cuivre(II)
dans laquelle R est choisi entre des groupes alkyle contenant 1 à 18 atomes de carbone, des groupes aryle contenant 6 à 12 atomes de carbone, des groupes alkoxy contenant 1 à 18 atomes de carbone, des groupes aryloxy contenant 6 à 12 atomes de carbone, ou bien dans laquelle R représente un groupe L-prolyl-L-valyl-L-phénylalanyl-L-valine ou L-valyl-L-phénylalanyl-L-valine, en association avec un support ou diluant acceptable en cosmétologie ou en dermatologie.

2. Composition de traitement cutané, comprenant du glycyl-(3)-méthyl)-L-histidyl-L-lysine:cuivre(II), en association avec un support ou diluant acceptable en cosmétologie et en dermatologie.

3. Composition comprenant du glycyl-(3-méthyl)-L-histidyl-L-lysine:cuivre(II), destinée à être utilisée dans un procédé pour accroître la quantité de graisse sous-cutanée chez des animaux à sang chaud.

4. Composition comprenant de la glycyl-(3-méthyl)-L-histidyl-L-lysine:cuivre(II), destinée à être utilisée dans un procédé pour accroître l'épaisseur du derme, de l'épiderme et des constituants sous-cutanés de la peau.

5. Composition comprenant du glycyl-(3-méthyl)-L-histidyl-L-lysine:cuivre(II), destinée à être utilisée dans un procédé pour accroître la densité dermique de la peau.

6. Composition comprenant du glycyl-(3-méthyl)-L-histidyl-L-lysine:cuivre(II), destinée à être utilisée dans un procédé pour accroître la vitesse de renouvellement des cellules dans l'épiderme humain.

7. Méthode de traitement cosmétique de la peau humaine, qui comprend l'application à la peau d'une composition comprenant du glycyl-(3-méthyl)-L-histidyl-L-lysine:cuivre(II).

8. Utilisation d'une composition comprenant du glycyl-(3-méthyl)-L-histidyl-L-lysine:cuivre(II) pour la production d'un médicament destiné à augmenter la quantité de graisse sous-cutanée chez des animaux à sang chaud.

9. Utilisation d'une composition comprenant un dérivé de GHL-Cu répondant à la formule générale :
(glycyl-L-histidyl-L-lysine-R):cuivre(II)
dans laquelle R est choisi entre des groupes alkyle contenant 1 à 18 atomes de carbone, des groupes aryle contenant 6 à 12 atomes de carbone, des groupes alkoxy contenant 1 à 18 atomes de carbone et des groupes aryloxy contenant 6 à 12 atomes de carbone, ou bien R représente un groupe L-prolyl-L-valyl-L-phénylalanyl-L-valine ou L-valyl-L-phénylalanyl-L-valine, pour la production d'un médicament destiné à accroître l'épaisseur du derme, de l'épiderme et des constituants souscutanés de la peau.

10. Utilisation d'une composition comprenant du glycyl-(3-méthyl)-L-histidyl-L-lysine:cuivre(II) pour la production d'un médicament destiné à accroître l'épaisseur du derme, de l'épiderme et des constituants sous-cutanés de la peau.

11. Utilisation d'une composition comprenant un dérivé de GHL-Cu répondant à la formule générale :
(glycyl-L-histidyl-L-lysine-R):cuivre (II)
dans laquelle R est choisi entre des groupes alkyle contenant 1 à 18 atomes de carbone, des groupes aryle contenant 6 à 12 atomes de carbone, des groupes alkoxy contenant 1 à 18 atomes de carbone et des groupes aryloxy contenant 6 à 12 atomes de carbone, ou bien R représente un groupe L-propyl-L-valyl-L-phénylalanyl-L-valine ou L-valyl-L-phénylalanyl-L-valine, pour la production d'un médicament destiné à accroître la densité dermique de la peau.

12. Utilisation d'une composition comprenant du (glycyl-L-histidyl-L-lysine-R):cuivre (II) pour la production d'un médicament destiné à accroître la densité dermique de la peau.

13. Utilisation d'une composition comprenant un dérivé de GHL-Cu répondant à la formule générale :
(glycyl-L-histidyl-L-lysine-R):cuivre(II)
dans laquelle R est choisi entre des groupes alkyle contenant 1 à 18 atomes de carbone, des groupes aryle contenant 6 à 12 atomes de carbone, des groupes alkoxy contenant 1 à 18 atomes de carbone et des groupes aryloxy contenant 6 à 12 atomes de carbone, ou bien R représente un groupe L-propyl-L-valyl-L-phénylalanyl-L-valine ou L-valyl-L-phénylalanyl-L-valine, pour la production d'un médicament destiné à accroître la vitesse de renouvellement des cellules de l'épiderme humain.

14. Utilisation d'une composition comprenant du glycyl-(3-méthyl)-L-histidyl-L-lysine:cuivre(II) pour la production d'un médicament destiné à accroître la vitesse de renouvellement des cellules de l'épiderme humain.
